# EUROPEAN PATENT SPECIFICATION

(11) **EP 3 094 208 B1**
(45) Date of publication and mention of the grant of the patent: **21.03.2018**
(21) Application number: 15701254.3
(22) Date of filing: 12.01.2015
(51) Int. Cl.: A45D 34/04, A45D 40/26, A61J 1/06, A61J 1/18, B65D 1/00, B65B 9/00

(54) **ROLL-ON CONTAINER FOR FLUID PRODUCTS, PARTICULARLY FOR MEDICAL, PHARMACEUTICAL, COSMETIC PRODUCTS OR THE LIKE**
AUFTRAGSROLLENBEHÄLTER FÜR FLÜSSIGPRODUKTE, INSBESONDERE FÜR MEDIZINISCHE, PHARMAZEUTISCHE, KOSMETISCHE UND ÄHNLICHE PRODUKTE
CONTENANT APPLICATEUR À ROULEAU POUR PRODUITS FLUIDES, EN PARTICULIER POUR PRODUITS MÉDICAUX, PHARMACEUTIQUES, COSMÉTIQUES OU SIMILAIRES

(30) Priority: 15.01.2014 IT MO20140006
(43) Date of publication of application: 23.11.2016
(73) Proprietor: Lameplast S.p.A., 41016 Rovereto sul Secchia - Novi Di Modena (MO) (IT)
(72) Inventor: FONTANA, Antonio, I-41012 Carpi (MO) (IT)
(74) Representative: Brunacci, Marco
(86) International application number: PCT/IB2015/050216
(87) International publication number: WO 2015/107445

(56) References cited:
- WO-A2-01/94213
- WO-A2-2007/107825
- DE-A1- 4 215 966

## Description

### Technical Field

The present invention relates to a roll-on container for fluid products, particularly for medical, pharmaceutical, cosmetic products or the like.

### Background Art

A known container for fluid products is known from WO01/94213. Containers are known which permit dispensing and distributing a fluid product - liquid, in paste or in powder - on a surface, by means of a so-called "roll-on" system.

The "roll-on" distribution system consists of a sphere engaged revolving in a sleeve which in turn is engaged, by pressure or screw, in the neck of a receptacle containing one or more doses of the product to be dispensed.

The sphere emerges and protrudes partially from the sleeve and can be covered and protected by means of a cap, which fits on the sleeve or on the neck of the receptacle.

The roll-on containers of known type have various drawbacks tied, in particular, to the fact that they are usually made of a plurality of components (at least four: the sphere, the sleeve, the receptacle and the cap).

Each component, in fact, must be inconveniently fabricated separately from the others, usually using plastic moulding methods requiring a dedicated mould for each component, with a cost that is not insubstantial.

To this must be added that a large number of components results in greater manufacturing problems and complications, relating to the need to assemble together the previously-fabricated parts.

It is therefore easy to appreciate that the production costs of the roll-on containers of traditional type are rather high and, inevitably, affect the retail price, with the risk of making the products less interesting for customers.

### Description of the Invention

The main aim of the present invention is to provide a roll-on container for fluid products, particularly for medical, pharmaceutical, cosmetic products or the like which is particularly cheap to fabricate and, at the same time, maintains all the functionality and practicality of use of traditional containers.

Another object of the present invention is to provide a roll-on container for fluid products, particularly for medical, pharmaceutical, cosmetic products or the like, which allows to overcome the mentioned drawbacks of the prior art within the ambit of a simple, rational, easy and effective to use solution.

The above mentioned objects are achieved by the present roll-on container for fluid products, particularly for medical, pharmaceutical, cosmetic products or the like, having the characteristics of claim 1, or by a strip of roll-on containers as defined by the features of claim 11.

### Brief Description of the Drawings

Other characteristics and advantages of the present invention will become better evident from the description of a preferred but not exclusive embodiment of a roll-on container for fluid products, particularly for medical, pharmaceutical, cosmetic products or the like, illustrated by way of an indicative, but not limitative, example in the accompanying drawings in which:
Figure 1 is an axonometric view, partially in vertical section, of a strip of containers according to the invention;
Figure 2 is an axonometric view of a container according to the invention during the opening phase;
Figure 3 is an axonometric view, on an enlarged scale and in vertical section, of a detail of the container according to the invention in the re-closing configuration.

### Embodiments of the Invention

With particular reference to these figures, globally indicated by reference numeral 1 is a roll-on container for fluid products, particularly for medical, pharmaceutical, cosmetic products or the like.

In this respect, it must be pointed out that within the scope of this treatise, by the term "fluid products" shall not be meant only liquid products, but also viscous products, e.g., in the state of paste or gel, and powdered products, in particular very fine powders with great fluidity.

In the embodiment shown in the illustrations, the containers 1 are designed to be fabricated and distributed on the market in the form of strips S, i.e., a group of containers 1 integral with one another and joined along specific breakable weakened areas 2, 3, better described below, which allow separating one container 1 from the other at the time of use.

The particular solution of producing containers 1 in the form of strips S makes the process of fabrication and distribution on the market even easier and further reduces the unit cost, fabricated container quantities being equal.

It is easy to appreciate however that alternative embodiments are possible wherein the containers 1 are made separately the one from the other.

Each container 1 comprises:
- at least a hollow body 4 which is designed for the containment of a fluid product F;
- at least a spherical distributor body 5, housed in a retaining seat 6 obtained in the hollow body 4 and able to allow the free rotation of the spherical distributor body 5;
- at least a closing body 7, 8, 9, able to cover at least partially the spherical distributor body 5.

The hollow body 4 is substantially tube-shaped and has:
- a first extremity 4a, in correspondence to which the retaining seat 6 is obtained; and
- a second extremity 4b, opposite the first extremity 4a, which is able to allow the introduction of the spherical distributor body 5 and of the fluid product F inside the hollow body 4 and can be closed after their introduction.

In other words, the hollow body 4 is initially fabricated so that the second extremity 4b is open, so as to allow access to the inner volume of the hollow body 4.

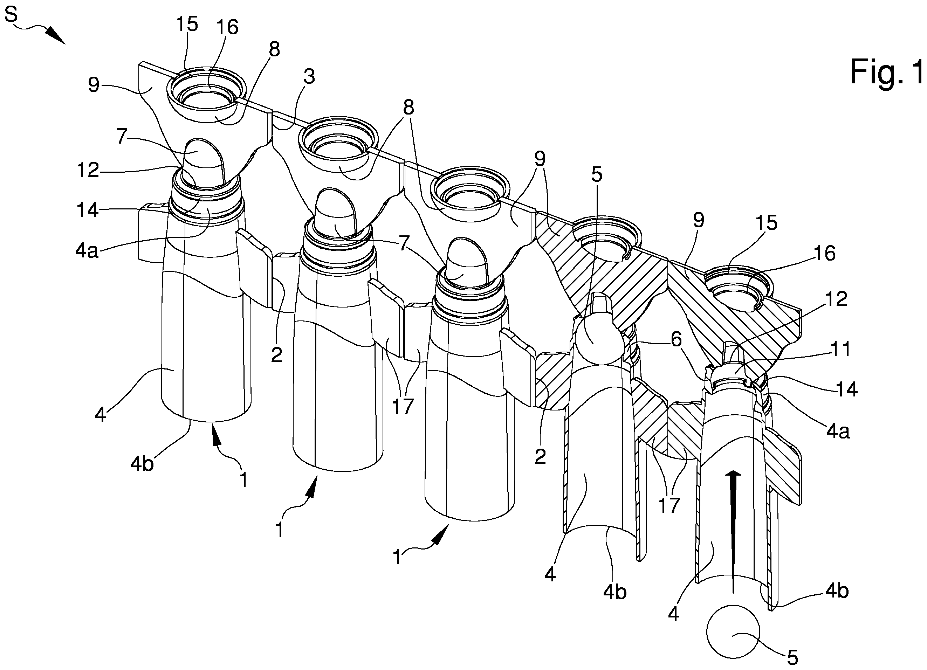

The introduction of the spherical distributor body 5 thus occurs through the second open extremity 4b to position it in the retaining seat 6 (figure 1).

In the same way, the hollow body 4 can be filled with the fluid product F through the second open extremity 4b.

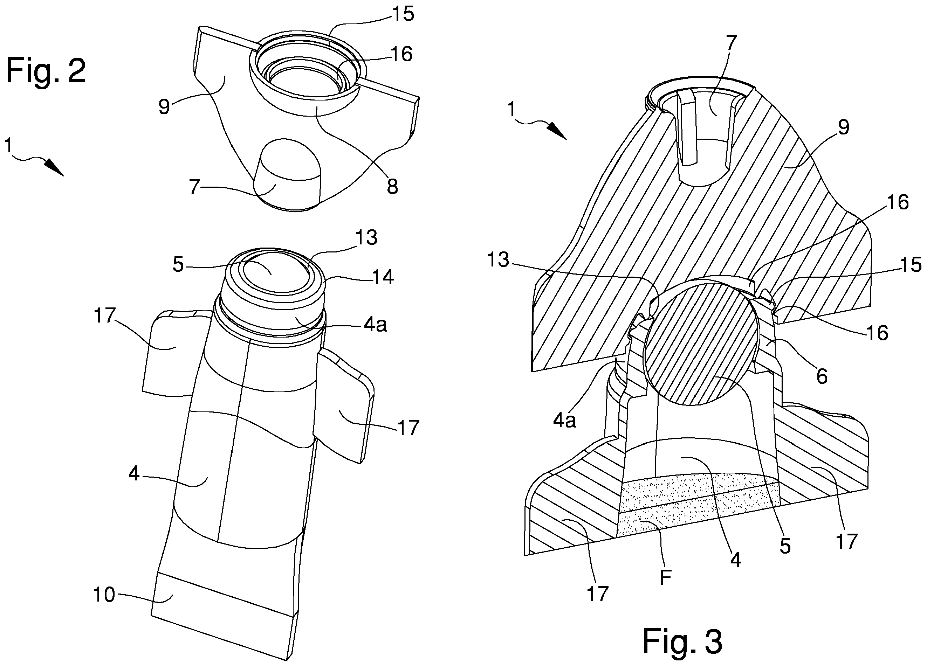

After filling, the second extremity 4b is closed, e.g., by crushing and sealing its rim, to obtain a sealed edge 10 visible in the figure 2.

Usefully, the retaining seat 6 consists in a circular sleeve section, having an inner surface 11 with a substantially spherical shape which complementarily reproduces a portion of the surface of the spherical distributor body 5.

The hollow body 4 and the closing body 7, 8, 9 of each container 1 are made in a single body piece and, in an initial configuration shown in figure 1, are joined along a pre-established breaking line 12 able to allow the separation of the closing body 7, 8, 9 from the hollow body 4 in an opening configuration (shown in figure 2) wherein on the hollow body 4, in correspondence to the pre-established breaking line 12, is obtained a mouth 13 from which the spherical distributor body 5 partially protrudes.

In other words, each container 1 consists only of two pieces:
- the single body piece defining both the hollow body 4 and the closing body 7, 8, 9; and
- the spherical distributor body 5.

The closing body 7, 8, 9 comprises at least a first covering portion 7, which is able to cover the spherical distributor body 5 in the initial configuration, and at least a second covering portion 8, which is apart from the first covering portion 7 and which, after the first opening of the container 1, can be fitted on the mouth 13 to cover the spherical distributor body 5 in a re-closing configuration shown in figure 3.

The first covering portion 7, for example, is shaped like an overturned cup and consists of a wall that extends from the pre-established breaking line 12 in the opposite direction to the hollow body 4.

The second covering portion 8, on the other hand, is shaped substantially like a hollow cap, with an ample circular base that can be fitted around the first extremity of the hollow body 4.

The first covering portion 7 and the second covering portion 8 are arranged on opposite sides of the closing body 7, 8, 9.

In particular, the closing body 7, 8, 9 comprises a pickup and support portion 9 with which are associated the first covering portion 7 and the second covering portion 8.

The pickup and support portion 9 is substantially sheet-shaped while the first covering portion 7 and the second covering portion 8 have a substantially axial-symmetric conformation which is common to both the first covering portion 7 and to the second covering portion 8 and which lies in the plane defined by the substantially sheet-shaped pickup and support portion 9.

Each container 1 comprises removable connection means 14, 15, 16 to join the second covering portion 8 to the hollow body 4 in the re-closing configuration.

The removable connection means 14, 15, 16, for example, comprise at least a first annular tooth 14, obtained on the hollow body 4 in the proximity of the mouth 13, and a second annular tooth 15, obtained on the second covering portion 8 and that can be fastened to the first annular tooth 14.

The first annular tooth 14, in particular, protrudes outwards from the first extremity 4a of the hollow body 4.

The second annular tooth 15, instead, protrudes inside the hollow cap defined by the second covering portion 8, in the proximity of its ample circular base. The removable connection means 14, 15, 16, moreover, comprise at least a spacer edge 16 obtained on the second covering portion 8 and able to go to rest against the hollow body 4 in the proximity of the mouth 13.

The spacer edge 16, for example, consists of an annular wall which protrudes inside the hollow cap defined by the second covering portion 8.

In practice, in the re-closing configuration, the closing body 7, 8, 9 fitted on the mouth 13 remains fastened onto the hollow body 4 inasmuch as:
- the second annular tooth 15 couples with the first annular tooth 14 and prevents the reciprocal moving away of the hollow body 4 and of the closing body 7, 8, 9;
- the spacer edge 16 rests on the first extremity 4a, around the mouth 13, and prevents any further approach of the hollow body 4 and the closing body 7, 8,9.

The hollow bodies 4 of the strip S also comprise at least a substantially protruding spacer fin 17 which is associated, along a first breakable weakened area 2, with the spacer fin 17 of another container 1 arranged adjacent, so as to form the strip S.

Similarly, the closing bodies 7, 8, 9 are associated along a second breakable weakened area 3 with the closing body 7, 8, 9 of another container 1 arranged adjacent to form the strip S.

In practice, all the hollow bodies 4 and all the closing bodies 7, 8, 9 of the strip S are made in a single body piece, with the hollow bodies 4 joined to the other hollow bodies 4 and the closing bodies 7, 8, 9 joined to the other closing bodies 7, 8, 9.

It is easy to appreciate however that alternative embodiments are possible wherein all the hollow bodies 4 and all the closing bodies 7, 8, 9 of the strip S continue to be made in a single body piece, but only the hollow bodies 4 are joined together along the first breakable weakened areas 2, or only the closing bodies 7, 8, 9 are joined together along the second breakable weakened areas 3.

## Claims

1. Roll-on container (1) for fluid products, particularly for medical, pharmaceutical, cosmetic products or the like, comprising:
- at least a hollow body (4) for the containment of a fluid product (F);
- at least a spherical distributor body (5), housed in a retaining seat (6) obtained in said hollow body (4) and able to allow the free rotation of said spherical distributor body (5);
- at least a closing body (7, 8, 9), able to cover at least partially said spherical distributor body (5);
**characterized by** the fact that:
- said hollow body (4) and said closing body (7, 8, 9) are made in a single body piece and, in an initial configuration, are joined along a pre-established breaking line (12) able to allow the separation of said closing body (7, 8, 9) from said hollow body (4) in an opening configuration wherein on said hollow body (4), in correspondence to said pre-established breaking line (12), is obtained a mouth (13) from which said spherical distributor body (5) partially protrudes; and
- said closing body (7, 8, 9) comprises at least a first covering portion (7), which is able to cover said spherical distributor body (5) in said initial configuration, and at least a second covering portion (8), apart from said first covering portion (7), which can be fitted on said mouth (13) and able to cover said spherical distributor body (5) in a re-closing configuration.

2. Container (1) according to claim 1, **characterized by** the fact that said first covering portion (7) and said second covering portion (8) are arranged on opposite sides of said closing body (7, 8, 9).

3. Container (1) according to claim 1 or 2, **characterized by** the fact that it comprises removable connection means (14, 15, 16) to join said second covering portion (8) to said hollow body (4) in said re-closing configuration.

4. Container (1) according to claim 3, **characterized by** the fact that said removable connection means (14, 15, 16) comprise at least a first annular tooth (14) obtained on said hollow body (4) and a second annular tooth (15) obtained on said second covering portion (8) and that can be fastened to said first annular tooth (14).

5. Container (1) according to claim 3 or 4, **characterized by** the fact that said removable connection means (14, 15, 16) comprise at least a spacer edge (16) obtained on said second covering portion (8) and able to go to rest against said hollow body (4) in the proximity of said mouth (13).

6. Container (1) according to one or more of the preceding claims, **characterized by** the fact that said second covering portion (8) is substantially cap-shaped.

7. Container (1) according to one or more of the preceding claims, **characterized by** the fact that said hollow body (4) is substantially tube-shaped and has:
- a first extremity (4a), in correspondence to which said retaining seat (6) is obtained; and
- a second extremity (4b), opposite said first extremity (4a), which is able to allow the introduction of said spherical distributor body (5) and of said fluid product (F) inside said hollow body (4) and which can be closed after their introduction.

8. Container (1) according to one or more of the preceding claims, **characterized by** the fact that said retaining seat (6) comprises a circular sleeve section, having a substantially spherical inner surface (11).

9. Container (1) according to one or more of the preceding claims, **characterized by** the fact that said hollow body (4) comprises at least a substantially protruding spacer fin (17) which is associated along a first breakable weakened area (2) with the spacer fin (17) of another container (1) arranged adjacent to form a strip (S) of containers (1).

10. Container (1) according to one or more of the preceding claims, **characterized by** the fact that said closing body (7, 8, 9) is associated along a second breakable weakened area (3) with the closing body (7, 8, 9) of another container (1) arranged adjacent to form a strip (S) of containers (1).

11. Strip (S) of roll-on containers (1) for fluid products, particularly for medical, pharmaceutical, cosmetic products or the like, **characterized by** the fact that it comprises a plurality of containers (1), each comprising:
- at least a hollow body (4) for the containment of a fluid product (F);
- at least a spherical distributor body (5), housed in a retaining seat (6) obtained in said hollow body (4) and able to allow the free rotation of said spherical distributor body (5);
- at least a closing body (7, 8, 9), able to cover at least partially said spherical distributor body (5);
wherein:
- said hollow bodies (4) and said closing bodies (7, 8, 9) are made in a single body piece, wherein said hollow bodies (4) are joined together along first breakable weakened areas (2) and/or said closing bodies (7, 8, 9) are joined together along second breakable weakened areas (3);
- in an initial configuration, said hollow body (4) and said closing body (7, 8, 9) of each of said containers (1) are joined along a pre-established breaking line (12) able to allow the separation of said closing body (7, 8, 9) from said hollow body (4) in an opening configuration wherein on said hollow body (4), in correspondence to said pre-established breaking line (12), is obtained a mouth (13) from which said spherical distributor body (5) partially protrudes; and
- said closing body (7, 8, 9) of each of said containers (1) comprises at least a first covering portion (7), which is able to cover the relevant spherical distributor body (5) in said initial configuration, and at least a second covering portion (8), apart from said first covering portion (7), which can be fitted on the relevant mouth (13) and able to cover the relevant spherical distributor body (5) in a re-closing configuration.

## Patentansprüche

1. Rollerbehälter (1) für Flüssigprodukte, insbesondere für medizinische, pharmazeutische, kosmetische oder ähnliche Produkte, umfassend:
- zumindest einen Hohlkörper (4) zum Aufnehmen eines Flüssigprodukts (F);
- zumindest einen kugelförmigen Verteilerkörper (5), der in einem Haltesitz (6) untergebracht ist, der in dem Hohlkörper (4) vorgesehen und in der Lage ist, die freie Drehbewegung des kugelförmigen Verteilerkörpers (5) zuzulassen;
- mindestens einen Verschlusskörper (7, 8, 9), der in der Lage ist, den kugelförmigen Verteilerkörper (5) zumindest teilweise zu bedecken;
**dadurch gekennzeichnet, dass**:
- der Hohlkörper (4) und der Verschlusskörper (7, 8, 9) als einstückiges Teil gefertigt sind und, in einer Ausgangskonfiguration, entlang einer vorher festgelegten Brechlinie (12) verbunden sind, die das Abtrennen des Verschlusskörpers (7, 8, 9) von dem Hohlkörper (4) in einer Öffnungskonfiguration zulässt, wobei an dem Hohlkörper (4), übereinstimmend mit der vorher festgelegten Brechlinie (12), eine Öffnung (13) vorgesehen ist, aus der der kugelförmige Verteilerkörper (5) teilweise ragt; und
- der Verschlusskörper (7, 8, 9) zumindest einen ersten Abdeckabschnitt (7), der in der Lage ist, den kugelförmigen Verteilerkörper (5) in der Ausgangskonfiguration zu bedecken, und getrennt von dem ersten Abdeckabschnitt (7) zumindest einen zweiten Abdeckabschnitt (8) umfasst, der an der Öffnung (13) angebracht werden kann und in der Lage ist, den kugelförmigen Verteilerkörper (5) in einer Wiederverschlusskonfiguration zu bedecken.

2. Behälter (1) nach Anspruch 1, **dadurch gekennzeichnet, dass** der erste Abdeckabschnitt (7) und der zweite Abdeckabschnitt (8) auf gegenüberliegenden Seiten des Verschlusskörpers (7, 8, 9) angeordnet sind.

3. Behälter (1) nach Anspruch 1 oder 2, **dadurch gekennzeichnet, dass** er lösbare Verbindungsmittel (14, 15, 16) zum Verbinden des zweiten Abdeckabschnitts (8) mit dem Hohlkörper(4) in der Wiederverschlusskonfiguration umfasst.

4. Behälter (1) nach Anspruch 3, **dadurch gekennzeichnet, dass** die lösbaren Verbindungsmittel (14, 15, 16) zumindest einen ersten ringförmigen Zahn (14), der an dem Hohlkörper (4) vorgesehen ist, und einen zweiten ringförmigen Zahn (15) umfassen, der an dem zweiten Abdeckabschnitt (8) vorgesehen ist und der an dem ersten ringförmigen Zahn (14) befestigt werden kann.

5. Behälter (1) nach Anspruch 3 oder 4, **dadurch gekennzeichnet, dass** die lösbaren Verbindungsmittel (14, 15, 16) zumindest einen Distanzrand (16) umfassen, der an dem zweiten Abdeckabschnitt (8) vorgesehen und in der Lage ist, an dem Hohlkörper (4) in der Nähe der Öffnung (13) zur Anlage zu kommen.

6. Behälter (1) nach einem oder mehreren der vorhergehenden Ansprüche, **dadurch gekennzeichnet, dass** der zweite Abdeckabschnitt (8) im Wesentlichen kappenförmig ist.

7. Behälter (1) nach einem oder mehreren der vorhergehenden Ansprüche, **dadurch gekennzeichnet, dass** der Hohlkörper (4) im Wesentlichen röhrenförmig ist und Folgendes aufweist:
- ein erstes Ende (4a), wobei in Übereinstimmung damit der Haltesitz (6) vorgesehen ist; und
- ein zweites Ende (4b), gegenüber dem ersten Ende (4a), das in der Lage ist, das Einbringen des kugelförmigen Verteilerkörpers (5) und des Flüssigprodukts (F) in den Hohlkörper (4) zuzulassen und das nach deren Einbringen geschlossen werden kann.

8. Behälter (1) nach einem oder mehreren der vorhergehenden Ansprüche, **dadurch gekennzeichnet, dass** der Haltesitz (6) einen kreisförmigen Hülsenabschnitt umfasst, der eine im Wesentlichen kugelförmige Innenfläche (11) aufweist.

9. Behälter (1) nach einem oder mehreren der vorhergehenden Ansprüche, **dadurch gekennzeichnet, dass** der Hohlkörper (4) zumindest einen im Wesentlichen vorragenden Distanzflügel (17) umfasst, der entlang einem ersten zerbrechbaren geschwächten Bereich (2) an den Distanzflügel (17) eines weiteren Behälters (1) angeschlossen ist, der angrenzend angeordnet ist, um einen Streifen (S) von Behältern (1) zu bilden.

10. Behälter (1) nach einem oder mehreren der vorhergehenden Ansprüche, **dadurch gekennzeichnet, dass** der Verschlusskörper (7, 8, 9) entlang einem zweiten zerbrechbaren geschwächten Bereich (3) an den Verschlusskörper (7, 8, 9) eines weiteren Behälters (1) angeschlossen ist, der angrenzend angeordnet ist, sodass ein Streifen (S) von Behältern (1) entsteht.

11. Streifen (S) von Rollerbehältern (1) für Flüssigprodukte, insbesondere für medizinische, pharmazeutische, kosmetische oder ähnliche Produkte, **dadurch gekennzeichnet, dass** er eine Vielzahl von Behältern (1) umfasst, jeweils umfassend:
- zumindest einen Hohlkörper (4) zum Aufnehmen eines Flüssigprodukts (F);
- zumindest einen kugelförmigen Verteilerkörper (5), der in einem Haltesitz (6) untergebracht ist, der in dem Hohlkörper (4) vorgesehen und in der Lage ist, die freie Drehbewegung des kugelförmigen Verteilerkörpers (5) zuzulassen;
- mindestens einen Verschlusskörper (7, 8, 9), der in der Lage ist, den kugelförmigen Verteilerkörper (5) zumindest teilweise zu bedecken;
wobei:
- die Hohlkörper (4) und die Verschlusskörper (7, 8, 9) als einstückiges Teil gefertigt sind, wobei die Hohlkörper (4) entlang von ersten zerbrechbaren geschwächten Bereichen (2) miteinander verbunden sind und/oder die Verschlusskörper (7, 8, 9) entlang von zweiten zerbrechbaren geschwächten Bereichen (3) miteinander verbunden sind;
- in einer Ausgangskonfiguration der Hohlkörper (4) und der Verschlusskörper (7, 8, 9) von jedem der Behälter (1) entlang einer vorher festgelegten Brechlinie (12) verbunden sind, die das Abtrennen des Verschlusskörpers (7, 8, 9) von dem Hohlkörper (4) in einer Öffnungskonfiguration zulässt, wobei an dem Hohlkörper (4), übereinstimmend mit der vorher festgelegten Brechlinie (12), eine Öffnung (13) vorgesehen ist, aus der der kugelförmige Verteilerkörper (5) teilweise ragt; und
- der Verschlusskörper (7, 8, 9) von jedem der Behälter (1) zumindest einen ersten Abdeckabschnitt (7), der in der Lage ist, den entsprechenden kugelförmigen Verteilerkörper (5) in der Ausgangskonfiguration zu bedecken, und getrennt von dem ersten Abdeckabschnitt (7) zumindest einen zweiten Abdeckabschnitt (8) umfasst, der an der entsprechenden Öffnung (13) angebracht werden kann und in der Lage ist, den entsprechenden kugelförmigen Verteilerkörper (5) in einer Wiederverschlusskonfiguration zu bedecken.

## Revendications

1. Contenant applicateur à rouleau (1) pour produits fluides, en particulier pour produits médicaux, pharmaceutiques, cosmétiques ou similaires, comprenant :
- au moins un corps creux (4) destiné à contenir un produit fluide (F) ;
- au moins un corps distributeur sphérique (5), logé dans un siège de retenue (6) obtenu dans ledit corps creux (4) et apte à permettre la libre rotation dudit corps distributeur sphérique (5) ;
- au moins un corps de fermeture (7, 8, 9), apte à recouvrir au moins partiellement ledit corps distributeur sphérique (5) ;
**caractérisé en ce que** :
- ledit corps creux (4) et ledit corps de fermeture (7, 8, 9) sont réalisés en une seule pièce et, dans une configuration initiale, sont solidaires l'un de l'autre le long d'une ligne de rupture préétablie (12) apte à permettre la séparation dudit corps de fermeture (7, 8, 9) dudit corps creux (4) dans une configuration d'ouverture dans laquelle sur ledit corps creux (4), en correspondance avec ladite ligne de rupture préétablie (12), est obtenue une bouche (13) depuis laquelle ledit corps distributeur sphérique (5) fait partiellement saillie ; et
- ledit corps de fermeture (7, 8, 9) comprend au moins une première portion de couverture (7), qui est apte à recouvrir ledit corps distributeur sphérique (5) dans ladite configuration initiale, et au moins une seconde portion de couverture (8), distincte de ladite première portion de couverture (7), pouvant être adaptée sur ladite bouche (13) et apte à recouvrir ledit corps distributeur sphérique (5) dans une configuration de refermeture.

2. Contenant (1) selon la revendication 1, **caractérisé en ce que** ladite première portion de couverture (7) et ladite seconde portion de couverture (8) sont agencées sur des côtés opposés dudit corps de fermeture (7, 8, 9).

3. Contenant (1) selon la revendication 1 ou 2, **caractérisé en ce qu'**il comprend des moyens de raccordement amovibles (14, 15, 16) pour raccorder ladite seconde portion de couverture (8) audit corps creux (4) dans ladite configuration de refermeture.

4. Contenant (1) selon la revendication 3, **caractérisé en ce que** lesdits moyens de raccordement amovibles (14, 15, 16) comprennent au moins une première dent annulaire (14) obtenue sur ledit corps creux (4) et une seconde dent annulaire (15) obtenue sur ladite seconde portion de couverture (8) et qui peut être fixée à ladite première dent annulaire (14).

5. Contenant (1) selon la revendication 3 ou 4, **caractérisé en ce que** lesdits moyens de raccordement amovibles (14, 15, 16) comprennent au moins un bord d'écartement (16) obtenu sur ladite seconde portion de couverture (8) et apte à aller reposer contre ledit corps creux (4) à proximité de ladite bouche (13).

6. Contenant (1) selon l'une ou plusieurs des revendications précédentes, **caractérisé en ce que** ladite seconde portion de couverture (8) est sensiblement en forme de calotte.

7. Contenant (1) selon l'une ou plusieurs des revendications précédentes, **caractérisé en ce que** ledit corps creux (4) est de forme sensiblement tubulaire et possède :
- une première extrémité (4a), en correspondance avec laquelle ledit siège de retenue (6) est obtenu ; et
- une seconde extrémité (4b), opposée à ladite première extrémité (4a), qui est apte à permettre l'introduction dudit corps distributeur sphérique (5) et dudit produit fluide (F) à l'intérieur dudit corps creux (4) et qui peut être fermée après leur introduction.

8. Contenant (1) selon l'une ou plusieurs des revendications précédentes, **caractérisé en ce que** ledit siège de retenue (6) comprend un tronçon de manchon circulaire, possédant une surface intérieure sensiblement sphérique (11).

9. Contenant (1) selon l'une ou plusieurs des revendications précédentes, **caractérisé en ce que** ledit corps creux (4) comprend au moins une ailette entretoise sensiblement en saillie (17) qui est associée le long d'une première zone fragilisée ruptible (2) à l'ailette entretoise (17) d'un autre contenant (1) agencé de manière adjacente pour former une bande (S) de contenants (1).

10. Contenant (1) selon l'une ou plusieurs des revendications précédentes, **caractérisé en ce que** ledit corps de fermeture (7, 8, 9) est associé le long d'une seconde zone fragilisée ruptible (3) au corps de fermeture (7, 8, 9) d'un autre contenant (1) agencé de manière adjacente pour former une bande (S) de contenants (1).

11. Bande (S) de contenants applicateurs à rouleau (1) pour produits fluides, en particulier pour produits médicaux, pharmaceutiques, cosmétiques ou similaires, **caractérisée en ce qu'**elle comprend une pluralité de contenants (1), chacun comprenant :
- au moins un corps creux (4) destiné à contenir un produit fluide (F) ;
- au moins un corps distributeur sphérique (5), logé dans un siège de retenue (6) obtenu dans ledit corps creux (4) et apte à permettre la libre rotation dudit corps distributeur sphérique (5) ;
- au moins un corps de fermeture (7, 8, 9), apte à recouvrir au moins partiellement ledit corps distributeur sphérique (5) ;
dans laquelle :
- lesdits corps creux (4) et lesdits corps de fermeture (7, 8, 9) sont réalisés en une seule pièce, dans laquelle lesdits corps creux (4) sont reliés entre eux le long de premières zones fragilisées ruptibles (2) et/ou lesdits corps de fermeture (7, 8, 9) sont reliés entre eux le long de secondes zones fragilisées ruptibles (3) ;
- dans une configuration initiale, ledit corps creux (4) et ledit corps de fermeture (7, 8, 9) de chacun desdits contenants (1) sont solidaires l'un de l'autre le long d'une ligne de rupture préétablie (12) apte à permettre la séparation dudit corps de fermeture (7, 8, 9) dudit corps creux (4) dans une configuration d'ouverture dans laquelle sur ledit corps creux (4), en correspondance avec ladite ligne de rupture préétablie (12), est obtenue une bouche (13) depuis laquelle ledit corps distributeur sphérique (5) fait partiellement saillie ; et
- ledit corps de fermeture (7, 8, 9) de chacun desdits contenants (1) comprend au moins une première portion de couverture (7), qui est apte à recouvrir le corps distributeur sphérique concerné (5) dans ladite configuration initiale, et au moins une seconde portion de couverture (8), distincte de ladite première portion de couverture (7), pouvant être adaptée sur la bouche concernée (13) et apte à recouvrir le corps distributeur sphérique concerné (5) dans une configuration de refermeture.
